# FASCICULE DE BREVET EUROPEEN

(11) **EP 1 096 933 B1**
(45) Date de publication et mention de la délivrance du brevet: **02.05.2003**
(21) Numéro de dépôt: 99929450.7
(22) Date de dépôt: 08.07.1999
(51) Int. Cl.: A61K 31/415, A61K 31/36

(54) **UTILISATION D'UN INHIBITEUR DU CYTOCHROME P450 EN ASSOCIATION AVEC UN-ANTIFONGIQUE POUR LE TRAITEMENT DES MYCOSES**
VERWENDUNG VON EINEM CYTOCHROM P450 HEMMER IN KOMBINATION MIT EINEM FUNGIZID ZUR BEHANDLUNG VON MYKOSEN
USE OF AN INHIBITOR OF CYTOCHROME P450 OPTIONALLY COMBINED WITH AN ANTIFUNGAL AGENT FOR TREATING MYCOSES

(30) Priorité: 10.07.1998 FR 9808946
(43) Date de publication de la demande: 09.05.2001
(73) Titulaire: AG INNOVATIONS PHARMA, Sainte Eulalie (Gironde) (FR)
(72) Inventeur: AUZERIE, Jack, F-33560 Sainte-Eulalie (FR)
(74) Mandataire: Thébault, Jean-Louis
(86) Numéro de dépôt international: FR9901670
(87) Numéro de publication internationale: WO00002557

(56) Documents cités:
- WO-A-94/17798
- US-A- 4 524 068

## Description

La présente invention est relative à l'utilisation d'un inhibiteur du cytochrome P45O, éventuellement en association avec un fongicide, dans le traitement des mycoses.

Les mycoses superficielles sont dues essentiellement à deux types de champignons: les levures et les dermatophytes. Ces affections peuvent siéger sur différentes parties du corps telles que la peau, les ongles, le cuir chevelu et les muqueuses. Elles peuvent, chez certains patients, et particulièrement chez des patients immunodéprimés, disséminer dans les tissus sous-jacents, puis dans le reste de l'organisme.

Les pathologies fongiques cutanées les plus répandues sont les candidoses, le pityriasis versicolore et les dermatophytoses.

Les dermatophytoses sont dues à trois genres de champignons: *Epidermophyton, Microsporum* et *Trichophyton.* Ces pathologies sont facilitées par des conditions chaudes et humides et une mauvaise hygiène. Elles sont endémiques dans certains pays notamment en Extrême-Orient et en Afrique. Elles sont également en progression chez les patients immunodéprimés souffrant du SIDA (Martindale 31 édition 1996, page 397).

Actuellement différents traitements existent, qui associent des soins locaux (solutions iodées) et des molécules antifongiques.

Parmi les antifongiques locaux disponibles on peut citer de façon non exhaustive les dérivés azotés (éconazole, kétoconazole) les dérivés triazolés comme le fluconazole, les acides gras comme les acides propionique et caprylique et d'autres produits tels que le ciclopirox.

Certaines infections locales répondent faiblement aux traitements topiques, en raison de régions du corps fortement kératinisées, donc peu accessibles en profondeur aux traitements (les ongles, les cheveux, le cuir chevelu, les pieds), et du fait de l'apparition de souches résistantes.

Des traitements systémiques par voie orale ou intraveineuse peuvent être prescrits mais ils sont longs, entraînent des effets secondaires sévères (céphalées, troubles gastro-intestinaux, réactions allergiques, troubles hépatiques, troubles hématologiques) et ne sont pas toujours efficaces.

Le pipéronyl butoxyde est une molécule connue pour ses activités insecticides. Il n'a néanmoins jamais été utilisé, à la connaissance de la demanderesse, comme principe actif dans des compositions pharmaceutiques antifongiques.

La demanderesse s'est attachée à trouver un traitement des mycoses applicable à des parties du corps fortement kératinisées et efficaces à l'encontre de souches de champignons présentant une résistance accrue aux antifongiques déjà connus.

Elle a en particulier trouvé qu'un inhibiteur du cytochrome P450, associé à des fongicides déjà connus, en améliore l'efficacité.

Elle a aussi trouvé qu'un de ces inhibiteurs présente en lui-même une activité antifongique.

La présente invention a donc pour objet un médicament comprenant l'association d'au moins une molécule ayant des propriétés antifongiques, ou fongicide, et d'au moins un inhibiteur du cytochrome P450. De manière avantageuse, le fongicide et l'inhibiteur du cytochrome P450 sont associés en quantités pharmacologiquement synergiques.

De manière préférentielle, un tel inhibiteur est le pipéronyl butoxyde. Il peut néanmoins être tout autre inhibiteur du cytochrome P450, pour autant qu'il présente en lui-même une action antifongique, ou synergise l'action antifongique d'autres molécules, tel que la cimétidine, l'érythromycine, les tétracyclines, la troléandomycine et la métyrapone.

Selon un mode de mise en oeuvre préférentiel de l'invention, le fongicide est une imidazole, ou une pyridone. Il peut néanmoins être toute autre molécule présentant une activité antifongique, susceptible d'être synergisée par l'association avec un inhibiteur du cytochrome P450.

Selon un mode particulièrement préféré de l'invention, la molécule antifongique est l'éconazole, qui est une molécule de la famille des imidazoles. Elle peut aussi être la cyclopyroxylamine, le fenticonazole, le fluconazole, l'isoconazole, le kétoconazole, ou encore le tinidazole.

Elle peut encore être un acide gras, tel que les acides propionique et caprylique.

L'invention est de plus relative à l'utilisation du pipéronyl butoxyde, non associé à une autre molécule présentant une activité antifongique, pour le traitement de mycoses.

Le pipéronyl butoxyde, ou une association d'un autre inhibiteur du cytochrome P450 et d'un fongicide, peuvent être utilisés pour traiter de manière générale les mycoses, et en particulier les mycoses superficielles résultant de l'implantation de champignons à la surface de la peau, des ongles, du cuir chevelu ou des muqueuses. Les mycoses traitées peuvent être les candidoses, le pityriasis versicolore et les dermatophytoses.

Le pipéronyl butoxyde seul est préférentiellement utilisé pour traiter les dermatophytoses.

Ces molécules peuvent être utilisées pour traiter le patient par un procédé comprenant les étapes suivantes:
- application du médicament, ou de la composition pharmaceutique, contenant le pipéronyl butoxyde, ou une association d'un inhibiteur du cytochrome P450 et d'un fongicide, sur la partie du corps atteinte par la mycose, et
- maintien en contact de la composition durant le temps nécessaire à une action antifongique efficace.

Ces étapes sont répétées aussi souvent et aussi longtemps que nécessaire.

Ces molécules sont appliquées sous la forme d'une composition pharmaceutique.

La présente invention a donc pour objet une composition pharmaceutique comprenant l'association, en quantités pharmacologiquement efficaces, d'au moins une molécule ayant des propriétés antifongiques et d'au moins un inhibiteur du cytochrome P450, et d'excipients pharmaceutiquement compatibles.

De manière avantageuse, les quantités d'inhibiteurs du cytochrome P450, tels que le pipéronyl butoxyde, sont comprises entre environ 1 mg et 100 mg d'inhibiteur par gramme de la composition.

Une telle composition peut éventuellement comprendre entre environ 1 mg et 100 mg de fongicide par gramme de composition.

Une telle composition peut être sous la forme d'une crème, d'un gel, d'un shampooing, d'un vernis à ongle, d'une poudre, d'ovules ou d'une lotion, ou sous toute autre forme, en fonction du mode d'application, et du lieu d'application souhaité.

La présente invention est enfin relative à l'utilisation d'une association d'au moins un inhibiteur du cytochrome P450, et d'au moins un fongicide pour la fabrication d'un médicament pour le traitement des mycoses.

Elle est enfin relative à l'utilisation du pipéronyl butoxyde, en tant que principe actif, pour la fabrication d'un médicament pour le traitement des mycoses.

Les médicaments et compositions pharmaceutiques selon la présente invention sont fabriqués par simple mélange d'au moins un inhibiteur du cytochrome P450, et en particulier du pipéronyl butoxyde, avec des excipients pharmaceutiquement compatibles, et éventuellement un ou plusieurs fongicides.

La présente invention est illustrée sans pour autant être limitée par les exemples suivants:

### EXEMPLE 1:

### Utilisation d'une association de pipéronyl butoxyde et de fongicide

L'association d'une part du pipéronyl butoxyde , et d'autre part de la cyclopiroxolamine, ou de l'éconazole a été testée.

Ces molécules ont été incorporées dans un milieu gélosé dit de Sabouraud (peptone 10g/l, glucose 10 g/l, gomme agar 15 g/l).

Des souches de diverses espèces de champignons ont été ensemencées par stries par la méthode de Stires.

Le pourcentage de croissance a été mesuré au cours du temps, pour des périodes allant jusqu'à 21 jours, pour certaines des souches testées.

Les quantités de pipéronyl butoxyde, et de cyclopyroxolamine utilisées sont identiques et sont de 10 ou 20 µg/ml dans le cas de l'association entre ces deux composés.

Dans le cas de l'association entre le pipéronyl butoxyde et l'éconazole, elles sont respectivement de 10 ou 20 µg/ml de pipéronyl butoxyde et de 1 µg/ml d'éconazole.

Des souches appartenant aux espèces suivantes ont été testées: *Microsporum canis, Trichophyton rubrum, Trichophyton interdigitale, Trichophyton mentagrophytes, Microsporum persicolor, Epidermophyton floccosum, Trichophyton violaceum et Trichophyton soudanense.*

Les résultats obtenus figurent dans les tableaux 1 et 2 ci-après.

L'ensemble des résultats montre que l'association entre l'inhibiteur, c'est-à-dire le pipéronyl butoxyde, et le fongicide, (cyclopyroxolamine ou éconazole), présente une activité très supérieure à celles obtenues lorsque les fongicides, le pipéronyl butoxyde sont testés isolément.

Il existe donc une synergie entre les activités du pipéronyl butoxyde et des fongicides.

### EXEMPLE 2

### Activité antifongique du pipéronyl butoxyde

L'activité du pipéronyl butoxyde seul a été testée à diverses concentrations, comme indiqué dans le tableau 1 sur le milieu de Sabouraud, dépourvu de tout autre fongicide.

Les résultats obtenus pour huit souches de diverses espèces de champignons sont reportés dans le tableau 3 .

Ils montrent que le pipéronyl butoxyde présente en lui-même une activité antifongique.

### EXEMPLE 3:

### Vernis à ongle selon l'invention.

Un vernis à ongle a été préparé en mélangeant les composés suivants, selon des méthodes connues de l'homme du métier.
Cyclopiroxolamine 8g
Pipéronyl butoxyde 8g
Alcool isopropylique 30g
GANTREZ ES 435 (copolymère d'éther méthylvinylique et de monobutylester d'acide maléique) 26,5 g

### EXEMPLE 4:

### Crème antifongique selon l'invention.

Une crème antifongique selon la présente invention a été préparée en mélangeant les composés suivants, selon les méthodes connues de l'homme du métier.
Cyclopiroxolamine 1 g
Pipéronyl butoxyde 1g
Alcool benzylique 1g
Alcool stéarylique 10 g
Alcool cétylique 10 g
Alcool myristylique 10 g
Huile de vaseline fluide 10g
Span 60,5 g
Tween 60,5g
Eau purifiée 47 g

### EXEMPLE 5:

### Gels antifongiques selon l'invention.

Un gel antifongique a été préparé en mélangeant les composés ci-après selon des méthodes connues de l'homme du métier.
Cyclopiroxolamine 1 g
Hydroxypropylcellulose 2g
Ethanol 5 g
Méthyl paraben 0,1 g
Propyl paraben 0,1 g
Eau qsp 100 g

### EXEMPLE 6

### Solution antifongique selon l'invention.

Une solution antifongique a été préparée en mélangeant les composés suivants selon des méthodes connues de l'homme du métier.
Cyclopiroxolamine 1 g
Propylène glycol 5 g
Alcool qsp 100 ml.

### EXEMPLE 7:

### Shampooing antifongique selon l'invention

Un shampooing selon la présente invention a été préparé par mélange, selon des méthodes connues de l'homme du métier, les composés suivants:
Cyclopiroxolamine 1 g
Sodium laurylethersulfate 5 g
Méthyl paraben 0,1 g
Propyl paraben 0,1 g
Chlorure de sodium 0,5 g
Eau qsp 100 ml

### EXEMPLE 8:

### Poudre antifongique pour application locale selon l'invention.

Une poudre antifongique pour application locale a été préparée en mélangeant, selon des méthodes connues de l'homme du métier, les composés suivants:
Cyclopiroxolamine 1g
Talc 98,5 g
Silice colloïdale 0,5 g.

Dans les exemples 3 à 8, la cypclopiroxolamine peut avantageusement être substituée par de l'éconazole, du fenticonazole, du fluconazole, de l'isoconazole, du kétoconazole, ou encore du tinidazole

**TABLEAU 1**

| EFFICACITE DE LA CYCLOPIROXOLAMINE, DU PIPERONYL BUTOXYDE ET DE L'ASSOCIATION DES DEUX PRODUITS SUR LE % DE CROISSANCE DE DIFFERENTES SOUCHES DE DERMATOPHYTES | | | | | | | | | | | | | | | |
|---|---|---|---|---|---|---|---|---|---|---|---|---|---|---|---|
| Produits : | Cyclopiroxolamine | | | | | Piperonyl. | | | | | Association | | | | |
| SOUCHES. | J0 | J4 | J6 | J10 | J14 | J0 | J4 | J6 | J10 | J14 | J0 | J4 | J6 | J10 | J14 |
| Micro. Caris * | 0 | 5 | 20 | 60 | 100 | 0 | 0 | 5 | 45 | 60 | 0 | 0 | 5 | 20 | 40 |
| Tric.Rubrum * | 0 | 0 | 50 | 80 | 100 | 0 | 0 | 20 | 35 | 50 | 0 | 0 | 0 | 15 | 30 |
| Tric.Interdigitale* | 0 | 5 | 10 | 80 | 90 | 0 | 5 | 25 | 60 | 70 | 0 | 0 | 5 | 40 | 50 |
| Tric.Mentagophytes* | 0 | 5 | 60 | 100 | 100 | 0 | 0 | 25 | 60 | 65 | 0 | 0 | 0 | 40 | 55 |
| Micro.Persicolor** | 0 | 0 | 30 | 75 | 100 | 0 | 0 | 20 | 40 | 75 | 0 | 0 | 0 | 10 | 60 |
| Epid.Flocosum** | 0 | 0 | 0 | 85 | 100 | 0 | 0 | 10 | 45 | 70 | 0 | 0 | 0 | 5 | 55 |
| Tric.Soudanese** | 0 | 0 | 0 | 30 | 60 | 0 | 0 | 10 | 10 | 25 | 0 | 0 | 0 | 0 | 0 |
| Tric.Violaceum** | 0 | 0 | 40 | 60 | 100 | 0 | 0 | 15 | 35 | 35 | 0 | 0 | 0 | 0 | 0 |

| | | | | | | | | | | | | | | | |
|---|---|---|---|---|---|---|---|---|---|---|---|---|---|---|---|
| * 20µg/ml. Concentrations équivalentes de cyclopiroxolamine et d'inhibiteur | | | | | | | | | | | | | | | |
| ** 10µg/ml. Concentrations équivalentes de cyclopiroxolamine et d'inhibiteur | | | | | | | | | | | | | | | |

**TABLEAU 2.**

| EFFICACITE DE L'ECONAZOLE DU PIPERONYL BUTOXYDE ET DE L'ASSOCIATION DES DEUX PRODUITS SUR LE % DE CROISSANCE DE DIFfERENTES SOUCHES DE DERMATOPHYTES. | | | | | | | | | | | | | | | |
|---|---|---|---|---|---|---|---|---|---|---|---|---|---|---|---|
| Souches | Econazole 1µg/ml | | | | | Piperonyl. | | | | | Association | | | | |
| | J0 | J4 | J6 | J10 | J14 | J0 | J4 | J6 | J10 | J14 | J0 | J4 | J6 | J10 | J14 |
| Micro. Canis * | 0 | 0 | 10 | 40 | 80 | 0 | 0 | 5 | 45 | 60 | 0 | 0 | 5 | 10 | 15 |
| Tric.Rubrum * | 0 | 0 | 20 | 60 | 70 | 0 | 0 | 20 | 35 | 50 | 0 | 0 | 0 | 10 | 20 |
| Tric.Interdigitale* | 0 | 5 | 5 | 40 | 60 | 0 | 5 | 25 | 60 | 70 | 0 | 0 | 0 | 10 | 20 |
| Tric.Mentagophytes* | 0 | 5 | 10 | 30 | 40 | 0 | 0 | 25 | 60 | 65 | 0 | 0 | 0 | 0 | 15 |
| Micro.Persicolor** | 0 | 0 | 0 | 15 | 50 | 0 | 0 | 20 | 40 | 75 | 0 | 0 | 0 | 0 | 10 |
| Epid.Flocosum** | 0 | 0 | 0 | 5 | 10 | 0 | 0 | 10 | 45 | 70 | 0 | 0 | 0 | 0 | 5 |
| Tric.Soudanese** | 0 | 0 | 0 | 30 | 60 | 0 | 0 | 10 | 10 | 25 | 0 | 0 | 0 | 0 | 0 |
| Tric.Violaceum** | 0 | 0 | 40 | 60 | 100 | 0 | 0 | 15 | 35 | 35 | 0 | 0 | 0 | 0 | 0 |

| | | | | | | | | | | | | | | | |
|---|---|---|---|---|---|---|---|---|---|---|---|---|---|---|---|
| *20µg/ml Piperonyl. | | | | | | | | | | | | | | | |
| **10 µg/ml Piperonyl. | | | | | | | | | | | | | | | |

**TABLEAU 3.**

| Efficacité antifongique du piperonyl butoxyde sur l'inhibition de la croissance de différentes souches de champignons. | | | | | | | | | | | | | | | |
|---|---|---|---|---|---|---|---|---|---|---|---|---|---|---|---|
| | J+4 | | | | | J+10 | | | | | J+14 | | | | |
| SOUCHES. | CONC.µg/ml | | | | | CONC.µg/ml | | | | | CONC.µg/ml | | | | |
| *DERMATOPHYTES.* | 0 | 2 | 10 | 20 | 50 | 0 | 2 | 10 | 20 | 50 | 0 | 2 | 10 | 20 | 50 |
| Micro. Canis | 100 | 20 | 15 | 0 | 0 | 100 | 80 | 60 | 45 | 0 | 100 | 100 | 80 | 60 | 0 |
| Tric.Rubrum | 100 | 15 | 10 | 0 | 0 | 100 | 90 | 60 | 35 | 0 | 100 | 100 | 75 | 50 | 0 |
| Tric.Interdigitale | 100 | 30 | 15 | 5 | 0 | 100 | 80 | 70 | 60 | 0 | 100 | 100 | 80 | 70 | 0 |
| Tric.Mentagophytes | 100 | 30 | 10 | 0 | 0 | 100 | 80 | 60 | 60 | 0 | 100 | 100 | 70 | 65 | 0 |
| Micro.Persicolor | 100 | 20 | 0 | 0 | 0 | 100 | 85 | 40 | 20 | 0 | 100 | 100 | 75 | 25 | 0 |
| Epid.Flocosum | 100 | 15 | 0 | 0 | 0 | 100 | 90 | 45 | 25 | 0 | 100 | 100 | 70 | 30 | 0 |
| Tric.Soudanese | 100 | 25 | 0 | 0 | 0 | 100 | 80 | 10 | 0 | 0 | 100 | 100 | 25 | 0 | 0 |
| Tric.Violaceum | 100 | 25 | 0 | 0 | 0 | 100 | 90 | 35 | 15 | 0 | 100 | 100 | 35 | 25 | 0 |

## Revendications

1. Médicament comprenant l'association d'au moins une molécule ayant des propriétés antifongiques et d'au moins un inhibiteur du cytochrome P450.

2. Médicament selon la revendication 1 **caractérisé en ce que** la molécule ayant des propriétés antifongiques et l'inhibiteur du cytochrome P450 sont associés en quantités pharmacologiquement synergiques.

3. Médicament selon l'une des revendications 1 et 2 **caractérisé en ce que** l'inhibiteur du cytochrome P450 est le pipéronyl butoxyde.

4. Médicament selon l'une des revendications 1 à 3 **caractérisé en ce que** la molécule ayant des propriétés antifongiques est une imidazole ou une pyridone.

5. Médicament selon l'une des revendications 1 à 4 **caractérisé en ce que** la molécule ayant des propriétés antifongiques est l'éconazole.

6. Utilisation d'une association d'au moins une molécule ayant des propriétés antifongiques et d'au moins un inhibiteur du cytochrome P450 pour la fabrication d'un médicament pour le traitement des mycoses.

7. Utilisation du pipéronyl butoxyde pour la fabrication d'un médicament pour le traitement des mycoses.

8. Utilisation selon l'une des revendications 6 et 7 **caractérisée en ce que** les mycoses sont superficielles.

9. Composition pharmaceutique comprenant l'association en quantités pharmacologiquement efficaces d'au moins une molécule ayant des propriétés antifongiques et d'au moins un inhibiteur du cytochrome P450, et d'excipients pharmaceutiquement compatibles.

10. Composition selon la revendication 9 **caractérisée en ce qu'**elle comprend entre 1 mg et 100 mg environ de pipéronyl butoxyde par gramme de composition.

11. Composition selon l'une des revendications 9 et 10 **caractérisée en ce qu'**elle comprend entre environ 1 mg et 100 mg de molécule ayant des propriétés antifongiques, par gramme de composition.

12. Composition selon l'une des revendications 9 à 11 **caractérisée en ce qu'**elle est sous la forme d'une crème, d'un gel, d'un shampooing, d'un vernis à ongle, d'une poudre, d'ovules ou d'une lotion.

## Patentansprüche

1. Medikament, das eine Kombination aus wenigstens einem Molekül, das fungizide Eigenschaften hat, und aus wenigstens einem Cytochrom-P450-Inhibitor enthält.

2. Medikament nach Anspruch 1, **dadurch gekennzeichnet, daß** das Molekül, das fungizide Eigenschaften hat, und der Cytochrom-P450-Inhibitor in pharmakologisch synergetischen Mengen vereinigt sind.

3. Medikament nach einem der Ansprüche 1 und 2, **dadurch gekennzeichnet, daß** der Cytochrom-P450-Inhibitor Piperonylbutoxid ist.

4. Medikament nach einem der Ansprüche 1 bis 3, **dadurch gekennzeichnet, daß** das Molekül, das fungizide Eigenschaften hat, ein Imidazol oder ein Pyridon ist.

5. Medikament nach einem der Ansprüche 1 bis 4, **dadurch gekennzeichnet, daß** das Molekül, das fungizide Eigenschaften besitzt, Econazol ist.

6. Verwendung einer Kombination aus wenigstens einem Molekül, das fungizide Eigenschaften hat, und aus wenigstens einem Cytochrom-P450-lnhibitor für die Herstellung eines Medikaments für die Behandlung von Mykosen.

7. Verwendung von Piperonylbutoxid für die Herstellung eines Medikaments für die Behandlung von Mykosen.

8. Verwendung nach einem der Ansprüche 6 und 7, **dadurch gekennzeichnet, daß** die Mykosen oberflächlich sind.

9. Pharmazeutische Zusammensetzung, die eine Kombination aus wenigstens einem Molekül, das fungizide Eigenschaften hat, und aus wenigstens einem Cytochrom-P450-lnhibitor in pharmakologisch wirksamen Mengen sowie pharmazeutisch kompatible Exzipienten umfaßt.

10. Zusammensetzung nach Anspruch 9, **dadurch gekennzeichnet, daß** sie Piperonylbutoxid in einer Menge im Bereich von etwa 1 mg bis 100 mg pro Gramm der Zusammensetzung enthält.

11. Zusammensetzung nach einem der Ansprüche 9 und 10, **dadurch gekennzeichnet, daß** sie das Molekül, das fungizide Eigenschaften hat, in einer Menge im Bereich von etwa 1 mg bis 100 mg pro Gramm der Zusammensetzung enthält.

12. Zusammensetzung nach einem der Ansprüche 9 bis 11, **dadurch gekennzeichnet, daß** sie in Form einer Crème, eines Gels, eines Shampoos, eines Nagellacks, eines Puders, von Zäpfchen oder einer Lotion vorliegt.

## Claims

1. A medicament comprising the association of at least one molecule having antifungal properties and at least one inhibitor of cytochrome P450.

2. A medicament according to claim 1, **characterised in that** the molecule having antifungal properties and the inhibitor of cytochrome P450 are associated in pharmacologically synergistic quantities.

3. A medicine according to one of claims 1 and 2, **characterised in that** the inhibitor of cytochrome P450 is piperonyl butoxide.

4. A medicine according to one of claims 1 to 3, **characterised in that** the molecule having antifungal properties is an imidazole or a pyridone.

5. A medicament according to one of claims 1 to 4, **characterised in that** the molecule having antifungal properties is econazole.

6. Use of an association of at least one molecule having antifungal properties and at least one inhibitor of cytochrome P450 for the manufacture of a medicament for the treatment of mycoses.

7. Use of piperonyl butoxide for the manufacture of a medicament for the treatment of mycoses.

8. Use according to one of claims 6 and 7, **characterised in that** the mycoses are superficial.

9. A pharmaceutical composition comprising the association in pharmacologically effective quantities of at least one molecule having antifungal properties and at least one inhibitor of cytochrome P450, and pharmaceutically compatible excipients.

10. A composition according to claim 9, **characterised in that** it comprises between 1 mg and 100 mg approximately of piperonyl butoxide per gram of composition.

11. A composition according to one of claims 9 and 10, **characterised in that** it comprises between approximately 1 mg and 100 mg of molecule having antifungal properties per gram of composition.

12. A composition according to one of claims 9 to 11, **characterised in that** it is in the form of a cream, a gel, a shampoo, a nail varnish, a powder, pessaries or a lotion.
